Europäisches Patentamt

European Patent Office

Office européen des brevets

(11) Veröffentlichungsnummer: **0 328 847**
**A1**

## EUROPÄISCHE PATENTANMELDUNG

(21) Anmeldenummer: 88810823.0

(51) Int. Cl.⁴: **A61F 2/34**

(22) Anmeldetag: 30.11.88

(30) Priorität: 14.01.88 CH 131/88

(43) Veröffentlichungstag der Anmeldung:
23.08.89 Patentblatt 89/34

(84) Benannte Vertragsstaaten:
AT DE FR GB IT

(71) Anmelder: GEBRÜDER SULZER AKTIENGESELLSCHAFT
Zürcherstrasse 9
CH-8401 Winterthur(CH)

Anmelder: Protek AG
Stadtbachstrasse 64
CH-3001 Bern(CH)

(72) Erfinder: Morscher, Erwin Walter, Prof. Dr.-med.
Orthopädische Universitätsklinik Basel
Felix Platter Spital CH-4055 Basel(CH)
Erfinder: Frey, Otto, Dr.
Wallrütistrasse 56
CH-8400 Winterthur(CH)

(54) Implantat zur Verstärkung des Randes eines Beckenknochens.

(57) In Verbindung mit einer künstlichen Hüftgelenkspfanne (3) ist im Bereich der Hauptbelastungsrichtung ein Implantat (4) am Rand des Beckens (1) implantiert, das die Form einer grabenförmigen Rinne (7) hat. Diese Rinne (7) ist mit verdichteten Bruchstücken aus spongiosem Gewebe (8) gefüllt.

Das Implantat (4) dient als Verstärkung des Beckenrandes im Bereich der Hauptbelastungsrichtung.

Fig.1

## Implantat zur Verstärkung des Randes eines Beckenknochens

Die Erfindung betrifft ein Implantat zur Verstärkung des Randes eines Beckenknochens, das zusammen mit einer künstlichen Hüftgelenkspfanne implantiert wird.

Bei Implantationen von künstlichen Hüftgelenkspfannen ist es häufig erforderlich, den Rand des Beckenknochens im Bereich der Hauptbelastungsrichtung durch zusätzliches Knochenmaterial zu verstärken. Bei einer bekannten Methode dieser sogenannten Spongiosaplastik werden relativ grosse spongiose Knochensplitter im Rand des Beckenknochens mit einzelnen Schrauben befestigt; hierbei erfolgt nur ein teilweises Zusammenwachsen der Knochensplitter untereinander und mit dem Beckenknochen, da ihre Ernährung und Versorgung nicht mehr gewährleistet ist. Darüberhinaus erfolgt ein Anpressen dieser Splitter und damit eine innige Verbindung mit dem Knochen bei Verwendung solcher grossen Splitter nur in der unmittelbaren Umgebung der Schrauben, so dass auch nur in diesem Bereich Verbindungen mit dem Beckenknochen entstehen.

Aufgabe der Erfindung ist es, das Verfahren der Spongiosaplastik zu verbessern. Diese Aufgabe wird mit der Erfindung dadurch gelöst, dass ein mehrlagiges, poröses Drahtnetz, das sich im Bereich der Hauptbelastungsrichtung über einen Teil eines Kreisumfanges erstreckt, zu einer grabenförmigen Rinne gebogen und mindestens über einen Teil seines Kreisumfanges zu einer Befestigungslasche verlängert ist, die mit Durchtrittsöffnungen für in den Knochen einzuschraubende Verankerungsschrauben versehen ist.

Das erfindungsgemässe Drahtnetz, das mit Vorteil intraoperativ durch bleibende Verformungen relativ genau an die individuellen Gegebenheiten angepasst werden kann, wird mit Hilfe von Knochenschrauben im Bereich der Hauptbelastungsrichtung am Becken befestigt; in seiner grabenförmigen Rinne können dann relativ kleinstückige Spongiosabruchteile eingelagert und verdichtet werden. Das kleinstückige verdichtete Material hat eine gute Chance, mit dem Beckenknochen zu verwachsen und ergibt so eine über den ganzen, von dem Drahtnetz überdeckten Umfangsbereich eine gleichmässige Verstärkung des Beckenrandes.

Das Drahtnetz, das mit Vorteil aus Titan oder einer Titanlegierung besteht, ist sowohl in Verbindung mit zementfrei implantierten Hüftgelenkspfanne als auch mit solchen anwendbar, die in einem Knochenzementbett fixiert werden.

Im folgenden wird die Erfindung anhand von Ausführungsbeispielen im Zusammenhang mit der Zeichnung näher erläutert.

Fig. 1 zeigt in einem Schnitt das an einem Beckenknochen verankerte Implantat in Verbindung mit einer rein schematisch angedeuteten künstlichen Hüftgelenkspfanne, die über ein Zementbett verankert ist;

Fig. 2 ist eine Ansicht von Fig. 1 von links unten in Richtung der Achse der Pfannenschale;

Fig. 3 zeigt, in gleicher Darstellung wie Fig. 1, die Anwendung des neuen Implantates in Verbindung mit einer zementfrei verankerten Hüftgelenkspfanne.

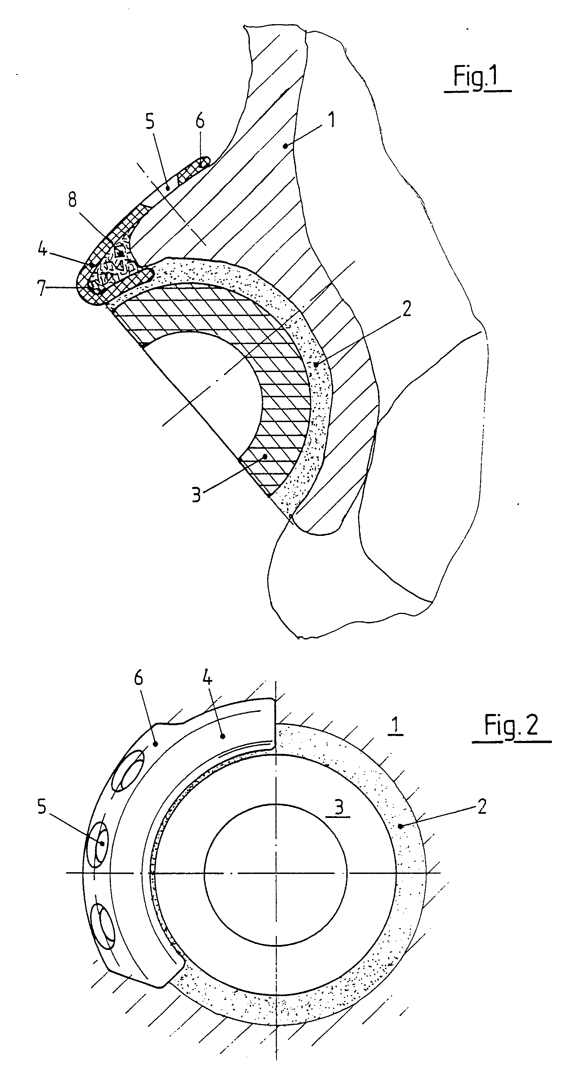

An einem Beckenknochen 1, in dem über ein Knochenzementbett 2 eine künstliche Hüftgelenkspfanne 3 verankert ist, ist im Bereich der Hauptbelastungsrichtung ein Verstärkungsimplantat 4 für den Beckenknochen fixiert. Dieses Implantat 4 wird beispielsweise durch nicht gezeigte Knochenschrauben gehalten, die durch Bohrungen 5 einer Befestigungslasche 6 (Fig. 2) des Implantates 4 hindurch in den Beckenknochen 1 eingeschraubt werden.

Das Implantat 4 erstreckt sich über einen Teil eines mit dem Pfannenkörper 3 konzentrischen Kreisumfanges. Es besteht aus einem mehrlagigen metallenen Drahtnetz, beispielsweise aus Titan oder einer Titanlegierung, das für eine individuelle Anpassung an einen Patentienten intraoperativ bleibend verformt werden kann. Der eigentliche "Funktionsteil" des Implantates 4 ist als V-förmiger Graben 7 an die Lasche 6 angesetzt. In diesem Graben 7 ist in relativ kleine Bruchstücke zerkleinerte Spongiosa 8 eingelagert und soweit wie möglich verdichtet, so dass ein Zusammenwachsen der Bruchstücke und eine innige Verbindung zum Beckenknochen 1 möglich ist.

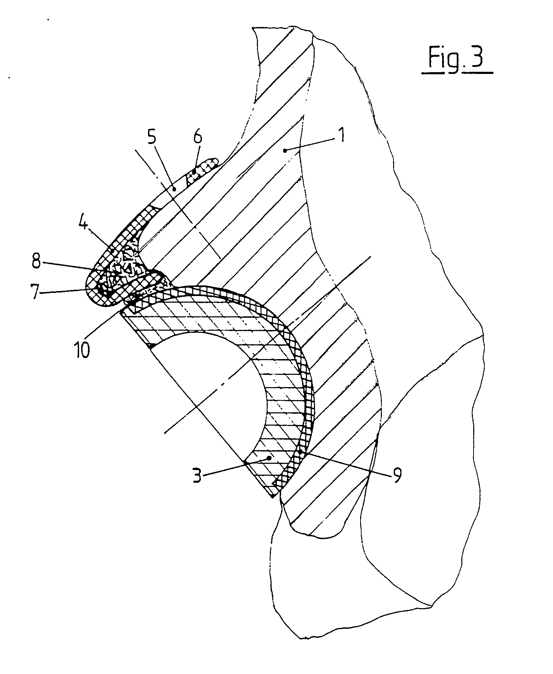

In Fig. 3 ist das Zementbett 2 durch eine poröse Metallgitterauflage 9 auf der aus Kunststoff bestehenden Pfanne 3 ersetzt. Bei einer derartigen zementfrei verankerten Pfanne 3 sind die beiden Drahtgitter 4 und 9 durch eine Zwischenschicht 10 aus Spongiosa-"Mus" getrennt; die Zwischenschicht 10 wird von der der Pfanne 3 zugewandten Seite her in das Gitter 4 nach dessen Montage eingepresst. Diese Zwischenschicht 10 schafft eine innige Verbindung zwischen den beiden Drahtgittern 4 und 9 aus revitalisiertem Knochengewebe.

## Ansprüche

1. Implantat zur Verstärkung des Randes eines Beckenknochens, das zusammen mit einer künstlichen Hüftgelenkspfanne implantiert wird, **gekennzeichnet durch** ein mehrlagiges, poröses Drahtnetz (4), das sich im Bereich der Hauptbelastungsrichtung über einen Teil eines Kreisumfanges erstreckt, zu einer grabenförmigen Rinne (7) gebogen und mindestens über einen Teil seines Kreisumfanges zu einer Befestigungslasche (6) verlängert ist, die mit Durchtrittsöffnungen (5) für in den Knochen (1) einzuschraubende Verankerungsschrauben versehen ist.

2. Implantat nach Anspruch 1, **dadurch gekennzeichnet, dass** es intraoperativ verformbar ist.

Fig.1

Fig.2

Fig. 3

Europäisches Patentamt

EUROPÄISCHER RECHERCHENBERICHT

Nummer der Anmeldung

EP 88 81 0823

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-A-3 007 548 (SARATOVSKIJ NAUCNO-ISSLEDOVATEL'SKIJ INSTITUT TRAVMATOLOGII I ORTOPEDII) * Figuren 1-3 * --- | 1,2 | A 61 F 2/34 |
| Y | FR-A-2 056 934 (C.F. THACKRAY LTD) * Figuren 1-3; Ansprüche 1-3 * --- | 1,2 | |
| A,P | WO-A-8 801 491 (S + G IMPLANTS GmbH) * Zusammenfassung * --- | 1 | |
| A | FR-A-2 595 241 (ERATO) * Zusammenfassung * ----- | 1 | |
| | | | **RECHERCHIERTE SACHGEBIETE (Int. Cl.4)** |
| | | | A 61 F |

Der vorliegende Recherchenbericht wurde für alle Patentansprüche erstellt

| Recherchenort | Abschlußdatum der Recherche | Prüfer |
|---|---|---|
| DEN HAAG | 26-04-1989 | ARGENTINI A. |